# FASCICULE DE BREVET EUROPEEN

(11) **EP 2 970 574 B1**
(45) Date de publication et mention de la délivrance du brevet: **22.02.2017**
(21) Numéro de dépôt: 14715359.7
(22) Date de dépôt: 12.03.2014
(51) Int. Cl.: C07C 69/708, C07C 59/305, C08G 64/02, C08G 64/42

(54) **OLIGOMÈRES ORGANIQUES DE GLYCÉROL ACYLÉS**
ORGANISCHE OLIGOMERE AUS ACYLIERTEM GLYCEROL
ORGANIC OLIGOMERS FROM ACYLATED GLYCEROL

(30) Priorité: 14.03.2013 FR 1352296
(43) Date de publication de la demande: 20.01.2016
(73) Titulaire: Agronutrition, 31390 Carbonne (FR); Institut National Polytechnique de Toulouse, 31400 Toulouse (FR); Institut National de la Recherche Agronomique (INRA), 75007 Paris (FR)
(72) Inventeur: MOULOUNGUI, Zéphirin, F-31000 Toulouse (FR); ABDEL BAKI, Zaher, F-31500 Toulouse (FR); VALENTIN, Romain, F-31400 Toulouse (FR); ZEBIB, Bachar, F-31280 Aigrefeuille (FR)
(74) Mandataire: Cabinet BARRE LAFORGUE & associés
(86) Numéro de dépôt international: PCT/FR2014/050564
(87) Numéro de publication internationale: WO 2014/140481

(56) Documents cités:
- FR-A1- 2 874 217
- FR-A1- 2 880 025

## Description

L'invention concerne des oligomères organiques de glycérol acylés. En particulier, l'invention concerne une nouvelle famille d'oligomères organiques de glycérol acylés susceptibles d'être obtenus par un procédé de synthèse simple, économique, et respectueux de l'environnement, notamment en une seule étape. L'invention concerne aussi une composition organique comprenant au moins un oligomère organique de glycérol acylé, et plus particulièrement une pluralité d'oligomères organiques de glycérol acylés.

On connaît déjà des oligomères de carbonate de glycérol partiellement acylés (FR 2 880 025) obtenus par acylation partielle d'oligomères de carbonate de glycérol. De tels oligomères partiellement acylés obtenus dans les exemples de FR 2 880 025 ne présentent pas au moins une extrémité de chaine principale de l'oligomère qui est acylée.

L'invention vise donc à proposer des oligomères organiques de glycérol acylés présentant au moins une -notamment deux- extrémité(s) de chaine principale qui est(sont) acylée(s).

L'invention vise aussi à proposer des oligomères organiques qui ne sont pas toxiques pour l'homme et pour son environnement, qui sont biodégradables et qui ne s'accumulent pas dans l'environnement après leur utilisation.

L'invention vise aussi en particulier à proposer des oligomères organiques pouvant être utilisés à titre d'adjuvants qui ne présentent pas de risques sanitaires pour l'homme et/ou l'animal.

L'invention vise aussi en particulier à proposer des oligomères organiques susceptibles d'être élaborés simplement et à partir de ressources qui ne sont pas des ressources fossiles et qui sont donc renouvelables.

L'invention vise aussi en particulier à proposer une famille d'oligomères organiques de glycérol acylés qui sont polyfonctionnels et compatibles -notamment miscibles- avec un milieu polaire et avec un milieu apolaire. L'invention vise donc des oligomères organiques de glycérol acylés qui sont par nature amphiphiles.

L'invention vise aussi en particulier à proposer une telle famille d'oligomères organiques de glycérol acylés qui sont susceptibles d'être utilisés à titre de lubrifiant. L'invention vise aussi en particulier à proposer une telle famille d'oligomères organiques de glycérol acylés qui sont susceptibles d'être utilisés à titre de d'agent mouillant d'une surface hydrophobe.

L'invention vise aussi en particulier à proposer une telle famille d'oligomères organiques de glycérol acylés qui sont susceptibles d'être utilisés à titre d'intrant en agriculture, en particulier à titre d'adjuvant pour le traitement de plantes.

L'invention vise aussi en particulier à proposer une telle famille d'oligomères organiques de glycérol acylés qui sont susceptibles d'être obtenus par synthèse chimique à partir de ressources naturelles -notamment de ressources végétales- qui sont renouvelables, en particulier à partir de coproduits de l'industrie oléo-chimique.

L'invention vise aussi en particulier à proposer une composition comprenant au moins un oligomère selon l'invention -notamment une composition organique comprenant une pluralité d'oligomères distincts selon l'invention- directement issue d'une synthèse de ces oligomères, et qui puisse être utilisée directement -notamment qui ne nécessite pas, pour son utilisation, d'étape de purification de chacun des oligomères organiques de glycérol acylés formés lors de la synthèse-.

L'invention vise également à proposer une telle famille d'oligomères organiques de glycérol acylés qui sont de faible coût de revient.

L'invention vise aussi en particulier à proposer une telle famille d'oligomères organiques de glycérol acylés susceptibles d'être obtenus lors de la mise en oeuvre d'un procédé de synthèse d'oligomères organiques de glycérol acylés en une seule étape.

Pour ce faire, l'invention concerne des oligomères organiques de glycérol acylés de formule (I) générale suivante : 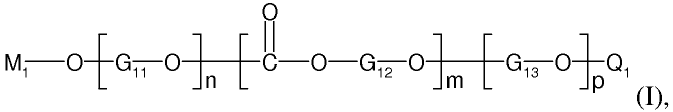 dans laquelle :
- M₁ est un groupement organique choisi dans le groupe formé des groupements de formules suivantes : 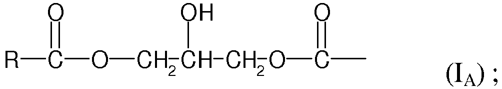 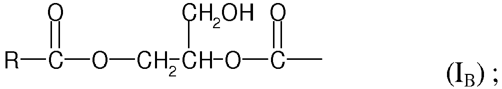 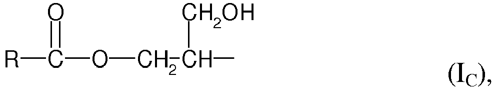 et; 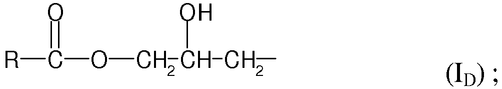 dans lesquels R est un groupement hydrocarboné -notamment un groupement hydrocarboné saturé, un groupement hydrocarboné insaturé ou un groupement hydrocarboné ramifié- comprenant de 1 à 21 atomes de carbone, et ;
- G₁₁ est choisi dans le groupe formé des groupements propyles hydroxylés de formules générales (II_{A}) et (II_{B}) suivantes : 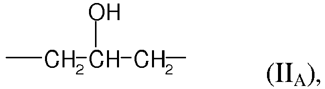 et ; 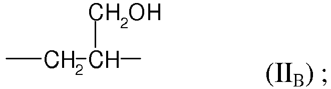
- G₁₂ et G₁₃ sont des groupements propyle α/α'-hydroxy-acylés de formule générale (III) suivante : 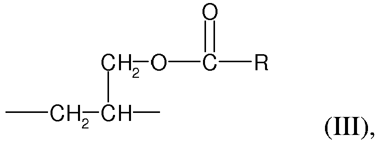 et;
- Q₁ est choisi dans le groupe formé de l'hydrogène et des groupements organiques formés d'au moins deux atomes liés par des liaisons covalentes et appartenant au groupe formé du carbone (C), de l'hydrogène (H) et de l'oxygène (O);
- n est un nombre entier naturel inférieur à 9 -c'est-à-dire de l'intervalle [0 ; 8]- tel que si n = 0 et m ≠ 0, alors M₁ est choisi dans le groupe formé de I_{C} et I_{D}, et ;
- m est un nombre entier naturel inférieur à 5 -notamment de l'intervalle [0 ; 4], et ;
- p est un nombre entier naturel inférieur à 4 -notamment de l'intervalle [0 ; 3]-.

Dans toute la suite, les groupements asymétriques G₁₁ de formule (II_{B}), G₁₂ et G₁₃ de formule (III) définissent l'un ou l'autre des deux sens d'insertion possible du groupement G₁₁ de formule (II_{B}) et des groupements G₁₂ et G₁₃ de formule (III) dans l'oligomère organique de glycérol acylé. Le groupement asymétriques G₁₁ de formule (II_{B}) définit l'une et l'autre des deux structures (a) et (b) générales suivantes :

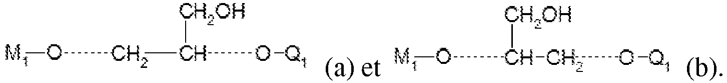

Les groupements asymétriques G₁₂ et G₁₃ de formule (III) définissent l'une et l'autre des deux structures (c) et (d) générales suivantes :

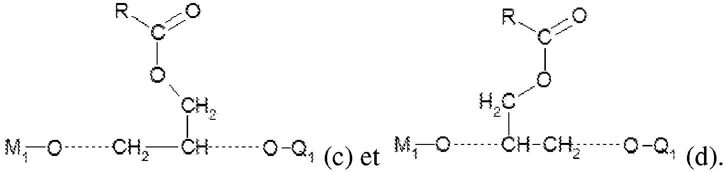

Le groupement G₁₁ de formule (II_{A}) peut présenter, dans l'oligomère organique de glycérol acylé, un sens unique d'orientation. Par exemple, tous les groupements propyle hydroxylés de formule (II_{A}) peuvent être orientés dans l'oligomère organique de glycérol acylé selon le sens (a). Tous les groupements propyle hydroxylés de formule (II_{A}) peuvent être orientés dans l'oligomère organique de glycérol acylé selon le sens (b). Les groupements propyle hydroxylés de formule (II_{A}) peuvent aussi être orientés dans l'oligomère organique de glycérol acylé régulièrement ou irrégulièrement selon le sens (a) ou selon le sens (b).

Les groupements G₁₂ et G₁₃ de formule (III) peuvent présenter, dans l'oligomère organique de glycérol acylé, un sens unique d'orientation. Par exemple, tous les groupements G₁₂ et G₁₃ de formule (III) peuvent être orientés dans l'oligomère organique de glycérol acylé selon le sens (c). Tous les groupements G₁₂ et G₁₃ de formule (III) peuvent en variante être orientés dans l'oligomère organique de glycérol acylé selon le sens (d). Les groupements G₁₂ et G₁₃ de formule (III) peuvent aussi être orientés dans l'oligomère organique de glycérol acylé régulièrement ou irrégulièrement selon le sens (c) ou selon le sens (d).

Les inventeurs ont observé qu'il est possible de synthétiser de tels oligomères organiques de glycérol acylés à partir de glycérol, d'au moins un précurseur choisi dans le groupe formé des esters carboniques cycliques de glycérol et des esters carboniques cycliques de glycérol α/α'-acylés et d'au moins un catalyseur métallique choisi dans le groupe formé du stéarate de zinc et du sulfate de zinc. On forme de tels oligomères organiques de glycérol acylés selon l'invention en mettant en contact chaque ester carbonique cyclique de glycérol, chaque ester carbonique cyclique de glycérol α/α'-acylé, chaque catalyseur métallique, du glycérol dans un réacteur, puis on ferme ledit réacteur de façon à former dans le réacteur une enceinte hermétiquement close aux gaz, puis on chauffe le mélange du(des) précurseur(s), du(des) catalyseur(s) métallique(s) et du glycérol de façon à ce que la température dans le réacteur atteigne une température de réaction comprise entre 150°C et 220°C et de façon à placer le mélange liquide sous pression, dite pression autogène, dans ledit réacteur, puis on ajuste la pression à l'intérieur du réacteur à une valeur comprise entre la valeur de la pression autogène et la pression atmosphérique pendant une durée nécessaire pour permettre la formation d'au moins un oligomère organique de glycérol acylé de formule (I) selon l'invention.

Il est donc possible d'obtenir de tels oligomères organiques de glycérol acylés qui sont des composés organiques polyfonctionnels, en particulier amphiphiles, formés de groupements sensiblement hydrophiles et de groupements hydrophobes par un procédé « one-pot » et en une seule étape et à partir de ressources naturelles, en particulier de ressources végétales.

Avantageusement et selon l'invention, Q₁ est choisi dans le groupe formé de l'hydrogène (H), des groupements propyles hydroxylés de formule (III_{A}) suivante : 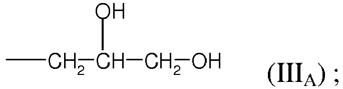 des groupements propyles hydroxylés de formule (III_{B}) suivante : 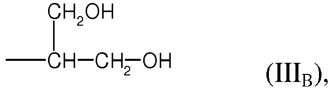 et; des groupements de formules (I_{A}), (I_{B}), (I_{C}) et (I_{D}).

Avantageusement et selon l'invention, lorsque m = p = 0 et M₁ est de formule (I_{A}), alors Q₁ est l'hydrogène (H). L'oligomère organique de glycérol acylé de formule (I) selon l'invention est un ester carbonique de glycérol acylé de formule (IV) générale suivante : 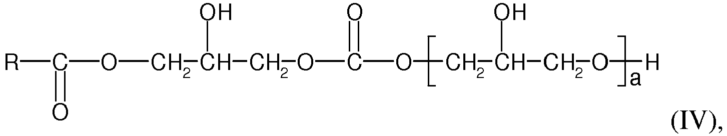 dans laquelle a est un nombre entier non nul inférieur à 9.

Avantageusement et selon l'invention, lorsque m = 0 et M₁ est de formule (I_{B}), alors Q₁ est l'hydrogène (H). L'oligomère organique de glycérol acylé de formule (I) selon l'invention est un ester carbonique de glycérol acylé de formule (V) générale suivante : 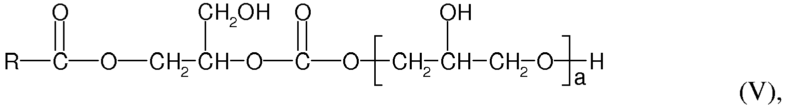 dans laquelle a est un nombre entier non nul inférieur à 9.

Avantageusement et selon l'invention, lorsque m = p = 0 et M₁ est de formule (I_{C}), alors Q₁ est un groupement de formule générale (I_{C}). L'oligomère organique de glycérol acylé de formule (I) selon l'invention est de formule (VI) générale suivante : 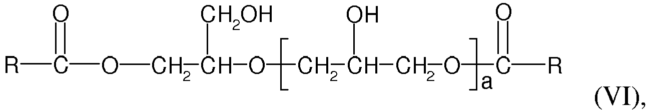 dans laquelle a est un nombre entier non nul inférieur à 4, notamment inférieur à 2.

On forme de tels oligomères organiques de glycérol acylés de formules (I), (IV), (V), (VI) ci-dessus ou de formules (VII), (VIII), (IX), (X), (XI), (XII), (en mettant en contact chaque ester carbonique cyclique de glycérol, chaque ester carbonique cyclique de glycérol α/α'-acylé, chaque catalyseur métallique et du glycérol dans un réacteur, puis on ferme ledit réacteur de façon à former dans le réacteur une enceinte hermétiquement close aux gaz, puis on chauffe le mélange du(des) précurseur(s), du(des) catalyseur(s) métallique(s) et du glycérol de façon à ce que la température dans le réacteur atteigne une température de réaction comprise entre 150°C et 220°C et de façon à placer le mélange liquide sous pression, dite pression autogène, dans ledit réacteur, puis on ajuste la pression à l'intérieur du réacteur à une valeur comprise entre la valeur de la pression autogène et la pression atmosphérique pendant une durée nécessaire pour permettre la formation d'au moins un oligomère organique de glycérol acylé de formule (I) selon l'invention.

Les inventeurs ont observé que, de façon avantageuse, l'ajustement -notamment par l'établissement d'une fuite de composition gazeuse du réacteur- de la pression à l'intérieur du réacteur à une valeur comprise entre la valeur de la pression autogène et la pression atmosphérique permet de contrôler la formation d'esters carboniques linéaires de glycérol α/α'-acylé.

En particulier, lorsque le mélange formé atteint la température de réaction et la pression autogène, on ouvre le réacteur de façon à diminuer la pression à l'intérieur du réacteur. Pour ce faire :
- on réalise une fuite de composition gazeuse du réacteur de façon à ajuster la pression dudit réacteur à une valeur de pression comprise entre la valeur de la pression atmosphérique et la valeur de pression autogène, atteinte dans le réacteur clos à la température de réaction, et ;
- on maintient la température de réaction pendant une durée adaptée pour permettre la formation d'au moins un oligomère organique de glycérol acylé selon l'invention.

Avantageusement, l'oligomère organique de glycérol acylé de formule (I) générale est un ester carbonique linéaire de glycérol α/α'-acylé.

Avantageusement, lorsqu'on utilise un catalyseur métallique zincique (par exemple ZnO, Zn(C₁₈H₃₅O₂)₂ et ZnSO₄), on obtient un mélange d'oligomères de formule (I) dans laquelle M₁ est de formule (I_{A}), (I_{B}), (I_{C}) ou (I_{D}), Q₁ est de formule (I_{A}), (I_{B}), (I_{C}), (I_{D}), G₁₁ ou H, n est un nombre entier naturel de l'intervalle [0 ; 8], m est un nombre entier naturel de l'intervalle [0 ; 4], p est un nombre entier naturel de l'intervalle [0 ; 3].

Avantageusement, lorsqu'on utilise un catalyseur métallique non zincique (FeSO₄, MnSO₄, CaCO₃ et Na₂CO₃), on obtient un mélange d'oligomères de formule (I) dans laquelle M₁ est de formule (I_{C}) ou (I_{D}), Q₁ est de formule (I_{C}), (I_{D}), G₁₁ ou H, n est un nombre entier naturel de l'intervalle [0 ; 8], m = 0, p est un nombre entier naturel de l'intervalle [0 ; 3].

Avantageusement et en variante selon l'invention, dans la formule (I) générale, M₁ et Q₁ sont de formule (I_{A}), G₁₁ est de formule (II_{A}), G₁₂ est de formule (III), n = 1, m est un nombre entier naturel de l'intervalle [1 ; 4] et p = 0. Un tel oligomère organique de glycérol acylé est en particulier un ester carbonique linéaire de glycérol α/α'-acylé de formule (VII) ci-après : 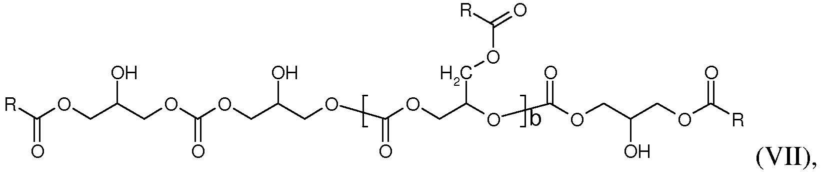 dans laquelle b est un nombre entier non nul inférieur à 5.

Avantageusement et en variante selon l'invention, dans la formule (I) générale, M₁ et Q₁ sont de formule (I_{A}), G₁₁ est de formule (II_{B}), G₁₂ est de formule (III), n = 1, m est un nombre entier naturel de l'intervalle [1 ; 4] et p = 0. Un tel oligomère organique de glycérol acylé est un ester carbonique linéaire de glycérol α/α'-acylé de formule (VIII) ci-après : 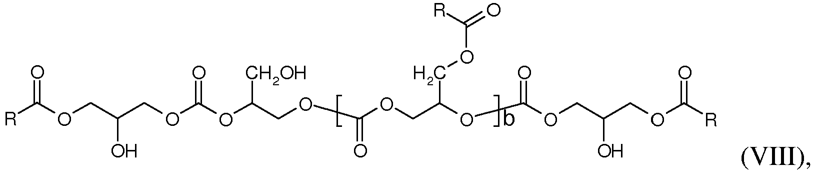 dans laquelle b est un nombre entier non nul inférieur à 5.

Avantageusement et en variante selon l'invention, dans la formule (I) générale, M₁ et Q₁ sont de formule (I_{A}), G₁₁ est de formule (II_{A}), G₁₂ est de formule (III), n = 1, m est un nombre entier naturel de l'intervalle [1 ; 4] et p = 0. Un tel oligomère organique de glycérol acylé est un ester carbonique linéaire de glycérol α/α'-acylé de formule (IX) ci-après : 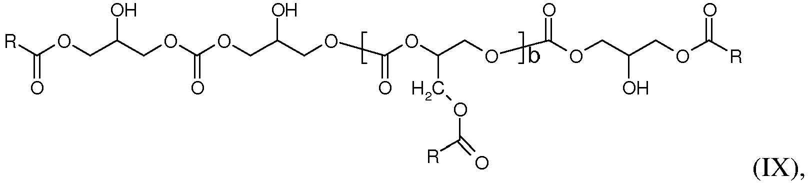 dans laquelle b est un nombre entier non nul inférieur à 5.

Avantageusement et en variante selon l'invention, dans la formule (I) générale, M₁ et Q₁ sont de formule (I_{A}), G₁₁ est de formule (II_{B}), G₁₂ est de formule (III), n = 1, m est un nombre entier naturel de l'intervalle [1 ; 4] et p = 0. Un tel oligomère organique de glycérol acylé est un ester carbonique linéaire de glycérol α/α'-acylé de formule (X) ci-après : 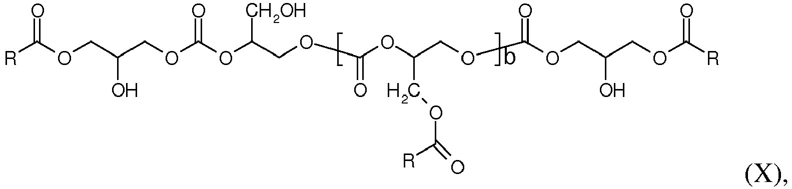 dans laquelle b est un nombre entier non nul inférieur à 5.

Avantageusement et en variante selon l'invention, dans la formule (I) générale, M₁ est de formule (I_{B}), Q₁ est de formule (I_{A}), G₁₁ est de formule (II_{A}), G₁₂ est de formule (III), n = 1, m est un nombre entier naturel de l'intervalle [1 ; 4] et p = 0. Un tel oligomère organique de glycérol acylé est un ester carbonique linéaire de glycérol α/α'-acylé de formule (XI) ci-après : 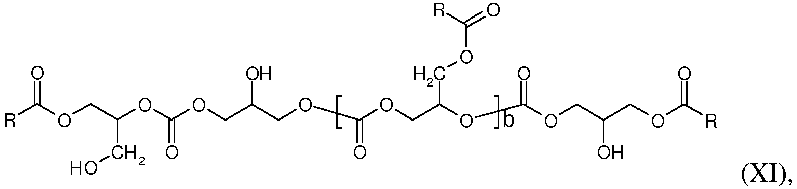 dans laquelle b est un nombre entier non nul inférieur à 5.

Avantageusement et en variante selon l'invention, dans la formule (I) générale, M₁ est de formule (I_{B}), Q₁ est de formule (I_{A}), G₁₁ est de formule (II_{B}), G₁₂ est de formule (III), n = 1, m est un nombre entier naturel de l'intervalle [1 ; 4] et p = 0. Un tel oligomère organique de glycérol acylé est un ester carbonique linéaire de glycérol α/α'-acylé de formule (XII) ci-après : 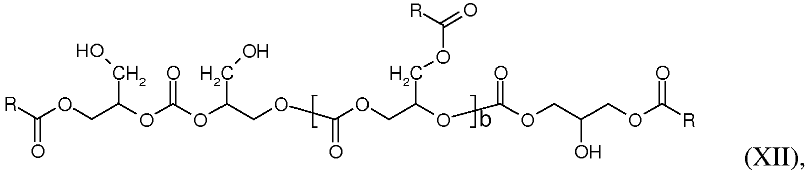 dans laquelle b est un nombre entier non nul inférieur à 5.

Avantageusement et en variante selon l'invention, dans la formule (I) générale, M₁ est de formule (I_{B}), Q₁ est de formule (I_{A}), G₁₁ est de formule (II_{A}), G₁₂ est de formule (III), n = 1, m est un nombre entier naturel de l'intervalle [1 ; 4] et p = 0. Un tel oligomère organique de glycérol acylé est un ester carbonique linéaire de glycérol α/α'-acylé de formule (XIII) ci-après : 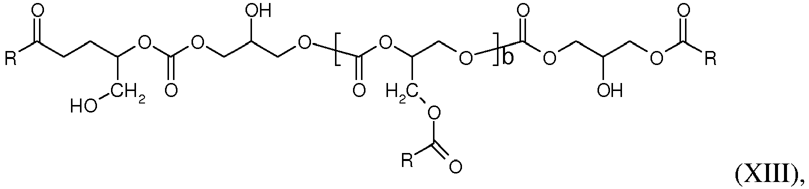 dans laquelle b est un nombre entier non nul inférieur à 5.

Avantageusement et en variante selon l'invention, dans la formule (I) générale, M₁ est de formule (I_{B}), Q₁ est de formule (I_{A}), G₁₁ est de formule (II_{B}), G₁₂ est de formule (III), n = 1, m est un nombre entier naturel de l'intervalle [1 ; 4] et p = 0. Un tel oligomère organique de glycérol acylé est un ester carbonique linéaire de glycérol α/α'-acylé de formule (XIV) ci-après : 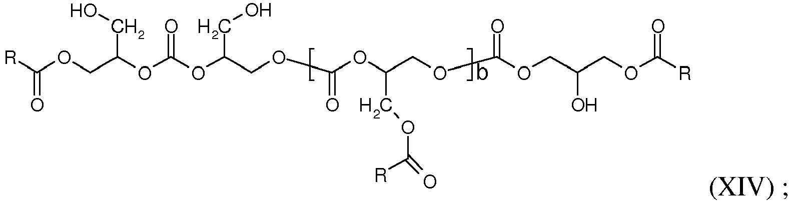 dans laquelle b est un nombre entier non nul inférieur à 5.

Avantageusement et selon l'invention, R est un groupement hydrocarboné choisi dans le groupe formé :
∘ des groupements hydrocarbonés saturés de formule générale -C_{q}H_{2q}+1 dans laquelle q est un nombre entier compris entre 1 et 21, et ;
∘ des groupements hydrocarbonés insaturés -notamment des groupements hydrocarbonés mono-insaturés de formule générale -CₛH₂ₛ₋₁ dans laquelle s est un nombre entier compris entre 1 et 21, et des groupements hydrocarbonés poly-insaturés-.

Avantageusement et selon l'invention, R est un groupement hydrocarboné choisi dans le groupe formé du méthyle (-CH₃), de l'éthyle (-CH₂-CH₃), du *n*-propyle (-CH₂-CH₂-CH₃), de l'*iso*-propyle (-CH(CH₃)₂), du *n*-butyle (-CH₂-CH₂-CH₂-CH₃), de l'*iso*-butyle (-CH₂-CH(CH₃)₂), du *tertio*-butyle (-C(CH₃)₃), du *n*-pentyle (-CH₂-(CH₂)₃-CH₃), de l'hexyle (-(CH₂)₅-CH₃), de l'octyle (-(CH₂)₇-CH₃), de l'undécyle (-(CH₂)₁₀-CH₃), du penta-décyle (-(CH₂)₁₄-CH₃), de l'hepta-décyle (-(CH₂)₁₆-CH₃) et de leurs équivalents insaturés -notamment du 9-ène-decyle (-CH=CH-(CH₂)₇-CH₃) et du 9-ène-heptadecyle (-(CH₂)₇-CH=CH-(CH₂)₇-CH₃).

Avantageusement et selon l'invention, l'oligomère organique de glycérol acylé de formule générale (I) présente une masse molaire supérieure à 400 g/mole.

L'invention concerne également une composition organique liquide comprenant au moins un oligomère organique de glycérol acylé selon l'invention. En particulier, elle concerne une composition organique liquide comprenant une pluralité d'oligomères organiques de glycérol acylés selon l'invention.

L'invention concerne également un oligomère organique de glycérol acylé et une composition organique liquide comprenant au moins un tel oligomère organique de glycérol acylé, caractérisés en combinaison par tout ou partie des caractéristiques mentionnées ci-dessus ou ci-après.

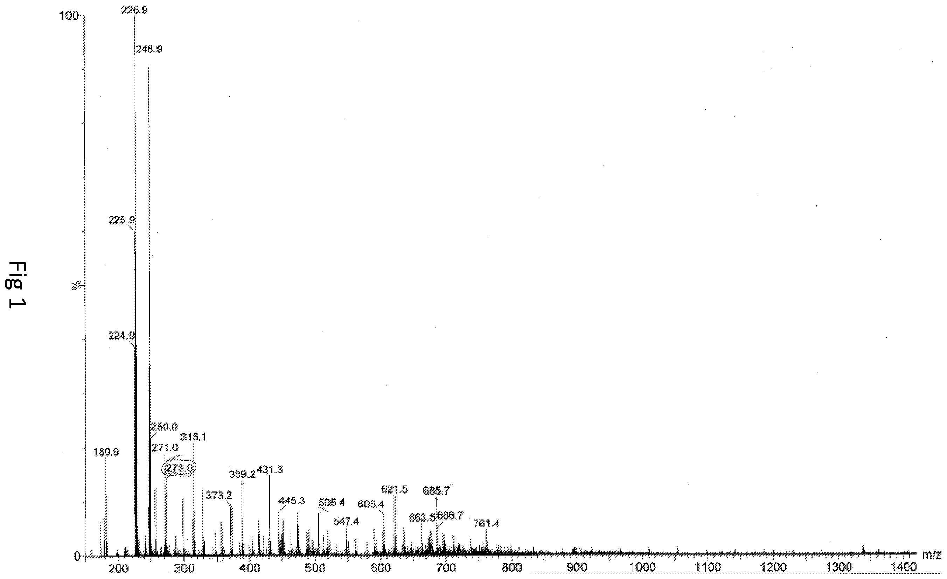

D'autres buts, caractéristiques et avantages de l'invention apparaîtront à la lecture de la description suivante et des exemples suivants donnés uniquement à titre non limitatif et dans lesquels la figure 1 est un spectrogramme de masse d'un milieu de réaction formé par la mise en oeuvre d'un procédé selon l'invention.

### EXEMPLE 1 - Synthèse d'esters carboniques de glycérol (cycliques) α/α'-acylés à titre de précurseur.

On réalise la synthèse d'esters carboniques de glycérol (cycliques) α/α'-acylés, en particulier de l'ester carbonique de glycérol α/α'-heptanoïque (ECG-C7), de l'ester carbonique de glycérol α/α'-nonanoïque (ECG-C9), de l'ester carbonique de glycérol α/α'-undécylénoïque (ECG-C11:1) et de l'ester carbonique de glycérol α/α'-oléique (ECG-C18:1) par estérification du carbonate de glycérol cyclique α/α'-hydroxylé par l'acide gras correspondant.

Dans un réacteur de 500 mL équipé d'un dispositif d'agitation mécanique, d'un dispositif de mise sous pression réduite et d'un dispositif « Dean-Stark » d'élimination de l'eau formée, on place 1,64 moles d'acide gras et 0,0078 moles d'acide 4-méthylbenzènesulfonique (CAS n° 6192-52-5, acide para-toluène-sulfonique, ApTs). On porte la température du mélange à la température de 110°C sous pression réduite de 800 hPa pendant une durée de 15 min. On ajoute ensuite goutte à goutte dans le réacteur 0,84 moles de carbonate de glycérol (cyclique) α/α'-hydroxylé sous agitation mécanique à 800 rotations par minutes (rpm) pendant 15 min. On place le réacteur dans un bain d'huile porté à la température de 110°C et sous agitation mécanique (800 rpm) pendant 3 heures.

### EXEMPLE 2 - Purification des esters carboniques de glycérol (cycliques) α/α'-acylés.

On dilue le milieu réactionnel dans 150 mL d'éther éthylique et on place le mélange obtenu dans une ampoule à décanter de 1 L. On lave le mélange successivement avec 4 volumes d'eau saturée en NaCl jusqu'à neutralité de la phase aqueuse. La phase organique lavée est séchée sur du sulfate de magnésium, puis est séparée du sulfate de magnésium hydraté par filtration. L'éther de la phase organique est éliminé par évaporation sous pression réduite. On obtient une masse de produit sec de 277g. L'ester carbonique de glycérol (cyclique) α/α'-acylé est séparé des acides gras en excès par distillation en film mince sous pression réduite (0,6 hPa) à une température inférieure à la température d'ébullition de l'acide gras sous cette pression réduite et inférieure à 155°C. On obtient l'ester carbonique de glycérol (cyclique) α/α'-acylé dont la pureté évaluée par chromatographie en phase gazeuse est comprise entre 85 % et 95 %.

### EXEMPLE 3 - Synthèse de l'ester carbonique de glycérol (cyclique) α/α'-acétylé (ECG-C2).

Dans un ballon tricol en verre de 2 L équipé d'un agitateur mécanique, d'un réfrigérant et placé dans un bain d'huile, on introduit 472 g de carbonate de glycérol cyclique (4-(hydroxyméthyle)-1,3-dioxolan-2-one, CAS 931-40-8) et 4 g de résine Lewatit K2431. On ajoute 6 moles d'anhydride acétique goutte à goutte dans le réacteur de façon à contrôler et maintenir la température du réacteur à 50°C sous agitation mécanique de 800 rpm pendant 4 heures.

On élimine l'excès d'anhydride acétique par évaporation à la température de 60°C et sous pression réduite de 55 hPa. On purifie l'ester carbonique linéaire de glycérol α/α'-acétylé par la technique du film mince conduite dans un évaporateur/séparateur à la température de 170°C et sous pression réduite de 0,33 hPa. On obtient l'ester carbonique de glycérol α/α'-acétylé dont la pureté évaluée par chromatographie en phase gazeuse est comprise entre 98 % et 99%.

Les caractéristiques structurelles des esters carboniques de glycérol (cycliques) α/α'-acylés obtenus aux exemples 1, 2 et 3 sont données au tableau 1 ci-après.

**Tableau 1**

| | Pureté, % | RMN ¹³C, ¹H | IRTF | Spectrométrie de masse, m/z |
|---|---|---|---|---|
| ECG-C2 | 98 | conforme | conforme | 160,1 |
| ECG-C7 | 94 | conforme | conforme | 230,2 |
| ECG-C9 | 95 | conforme | conforme | 258,3 |
| ECG-C11 :1 | 85 | conforme | conforme | 284,3 |
| ECG-C18 :1 | 96 | conforme | conforme | 382,5 |

### EXEMPLE 4 - Oligomérisation de l'ester carbonique de glycérol (cyclique) α/α'-acétylé (ECG-C2).

On réalise l'oligomérisation de l'ester carbonique de glycérol α/α'-acétylé (ECG-C2) obtenu à l'exemple 3 en présence d'un catalyseur métallique, de glycérol à titre d'amorceur organique, et dans les conditions décrites au tableau 2 ci-après.

**Tableau 2**

| Masse ECG-C2, g | Catalyseur métallique | | Amorceur Glycérol, g | Conditions | TC, % | Masses molaires en nombre |
|---|---|---|---|---|---|---|
| | Nature | Masse, mg | | | | |
| 4,25 | Stéarate de zinc Zn(C₁₈H₃₅O₂)₂ | 25 | 1,5 | 160°C, Pₐₜₘ, 2h | 59 | 393, 195, 64 |
| 8,5 | Zn(C₁₈H₃₅O₂)₂ | 25 | 1,5 | 160°C, Pₐₜₘ, 2h | 95 | 970, 688, 352, 274, 202, 190 |
| 42,5 | Zn(C₁₈H₃₅O₂)₂ | 250 | 7,5 | 160°C, 1800 hPa, 2h | 61 | 321, 157, 91 |
| 21,25 | Zn(C₁₈H₃₅O₂)₂ | 125 | 3,75 | 160°C, Pₐₜₘ, 2h | 84 | 714,336, 206, 139, 92 |
| 21,5 | Zn(C₁₈H₃₅O₂)₂ | 125 | 3,75 | 160°C, Pₐₜₘ, 2h | 99,2 | 405, 253, 172,93 |
| 21,5 | Zn(C₁₈H₃₅O₂)₂ | 125 | 3,75 | 160°C, Pₐₜₘ, 2h | 98,4 | 574,320, 160,95 |
| 21,5 | Zn(C₁₈H₃₅O₂)₂ | 125 | 3,75 | 160°C, Pₐₜₘ, 2h | 98,4 | 395, 189, 140,92 |
| 8,5 | ZnSO₄ | 50 | 1,75 | 160°C, 3400 hPa, 2h | 76 | 420, 173, 122, 84, 36 |
| 9 | ZnSO₄ | 50 | 1,7 | 160°C, Pₐₜₘ, 30h | 55 | 431, 160, 83 |
| 21,25 | ZnSO₄ | 125 | 3,75 | 160°C, Pₐₜₘ, 2h | 66 | 389, 159, 83 |
| 21,5 | FeSO₄ 8H₂O | 60 | 3,75 | 160°C, Pₐₜₘ, 2h | 40,3 | 446, 162, 96 |
| 21,5 | ZnO | 55 | 3,75 | 160°C, Pₐₜₘ, 2h | 46 | 322, 156, 95 |
| 21,5 | MnSO₄, 1H₂O | 118 | 3,75 | 160°C, Pₐₜₘ, 2h | 51 | 327, 156, 96 |
| 21,5 | MnSO₄, 1H₂O | 85 | 3,75 | 160°C, Pₐₜₘ, 2h | 34 | 323, 154, 94 |
| 21,5 | ZnSO₄, 1H₂O | 125 | 3,75 | 160°C, Pₐₜₘ, 2h | 36 | 327, 157, 94 |
| 21,5 | CaCO₃ | 70 | 3,75 | 160°C, Pₐₜₘ, 2h | 31 | 324, 154, 94 |
| 21,5 | Na₂CO₃ | 74 | 3,75 | 160°C, Pₐₜₘ, 2h | 98 | 632,314, 192, 137, 99 |

La valeur TC (%) indique le taux de conversion de l'ester carbonique de glycérol (cyclique) α/α'-acylé de départ. Les valeurs de masse molaire en nombre sont obtenues par analyse du milieu de réaction en chromatographie par perméation de gel sur colonne PLgel 3 µm MIXED-E. Les oligomères organiques de glycérol acylés sont détectés en sortie de colonne par réfractométrie et les masses moléculaires en nombre sont déterminées par comparaison avec des standards de polystyrène.

Les synthèses réalisées avec un catalyseur métallique zincique (ZnO, Zn(C₁₈H₃₅O₂)₂ et ZnSO₄) conduisent à un mélange d'oligomères de formule (I) dans laquelle M₁ est de formule (I_{A}), (I_{B}), (I_{C}) ou (I_{D}), Q₁ est de formule (I_{A}), (I_{B}), (I_{C}), (I_{D}), G₁₁ ou H, n est un nombre entier naturel de l'intervalle [0 ; 8], m est un nombre entier naturel de l'intervalle [0 ; 4], p est un nombre entier naturel de l'intervalle [0 ; 3] et R est un méthyle.

Un exemple de spectre de masse d'un milieu de réaction obtenu par mise en oeuvre d'un procédé d'oligomérisation de l'ester carbonique de glycérol α/α'-acétylé (ECG-C2) tel que décrit à l'exemple 4 est représenté en figure 1. Sont détectés des signaux correspondant à des ions moléculaires et fragments de valeurs m/z comprises entre 180,9 et 761,4 et correspondant à des oligomères organiques de glycérol de formules particulières (A), (B) et (C) suivantes : 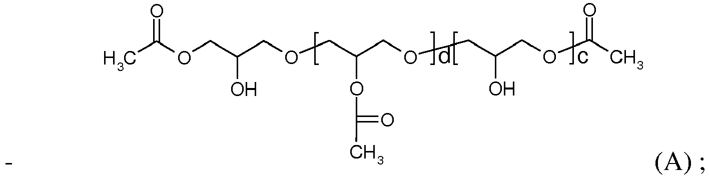 dans laquelle c peut prendre la valeur 1, 2 ou 4 et d peut prendre la valeur 1, 2, 3 ou 4; 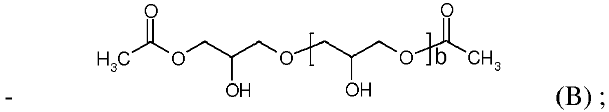 dans laquelle b peut prendre la valeur 1, 2, ou 3; 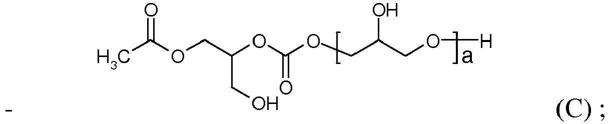 dans laquelle a peut prendre la valeur 1, 2, 3, 4, 5, 6, 7 ou 8, et ; 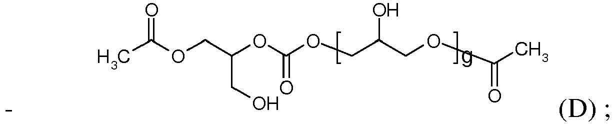 dans laquelle g peut prendre la valeur 1, 2, 3, 4, 5, 6, 7, 8, 9 ou 10.

Les synthèses réalisées avec un catalyseur métallique non zincique (FeSO₄, MnSO₄, CaCO₃ et Na₂CO₃) conduisent à un mélange d'oligomères de formule (I) dans laquelle M₁ est de formule (I_{C}) ou (I_{D}), Q₁ est de formule (I_{C}), (I_{D}), G₁₁ ou H, n est un nombre entier naturel de l'intervalle [0 ; 8], m = 0, p est un nombre entier naturel de l'intervalle [0 ; 3] et R est un méthyle.

EXEMPLE 5 - Oligomérisation des esters carboniques de glycérol (cycliques) α/α'-acylés (ECG-C2, ECG-C7, ECG-C9, ECG-C11:1 et ECG-C18:1).

On réalise une oligomérisation des esters carboniques de glycérol (cycliques) α/α'-acylés dans les conditions décrites au tableau 3 ci-après.

**Tableau 3**

| ECG, masse | Catalyseur métallique | | Amorceur Glycérol, g | Conditions | TC, % | Masses molaires en nombre |
|---|---|---|---|---|---|---|
| | Nature | Masse, mg | | | | |
| ECG-C2, 21,25 g | Zn(C₁₈H₃₅O₂)₂ | 125 | 3,75 | 160°C, Pₐₜₘ, 2h | 98 | 714,335, 206, 139, 92 |
| ECG-C2, 21,25 g | ZnSO₄ | 125 | 3,75 | 160°C, Pₐₜₘ, 2h | 66 | 389, 159, 83 |
| ECG-C7, 20 g | ZnSO₄ | 113 | 2,42 | 200°C, Pₐₜₘ, 2h | 64 | 639, 420, 252,96 |
| ECG-C9, 20 g | ZnSO₄ | 110 | 2,15 | 180°C, Pₐₜₘ, 2h | 88 | 992,541, 303, 90 |
| ECG-C11:1, 10g | ZnSO₄ | 86 | 1,3 | 190°C, Pₐₜₘ, 2h | 98 | 7632, 2113,1084, 750,486 |
| ECG-C18:1, 10g | ZnSO₄ | 50 | 0,7 | 200°C, Pₐₜₘ, 2h | 97 | 3724, 1787, 1169,613, 94 |

On obtient des oligomères de masse molaire apparente pouvant atteindre 7600 Da.

Les synthèses réalisées avec un catalyseur métallique zincique (Zn(C₁₈H₃₅O₂)₂ et ZnSO₄) conduisent à un mélange d'oligomères de formule (I) dans laquelle M₁ est de formule (I_{A}), (I_{B}), (I_{C}) ou (I_{D}), Q₁ est de formule (I_{A}), (I_{B}), (I_{C}), (I_{D}), G₁₁ ou H, n est un nombre entier naturel de l'intervalle [0 ; 8], m est un nombre entier naturel de l'intervalle [0 ; 4], p est un nombre entier naturel de l'intervalle [0 ; 3] et R est choisi parmi le méthyle (-CH₃), l'hexyle (-(CH2)₅-CH₃), l'octyle (-(CH2)₇-CH₃), le 9-ène-décyle (-CH=CH-(CH2)₇-CH₃) et le 9-ène-heptadécyle (-(CH2)₇-CH=CH-(CH2)₇-CH₃).

La valeur TC (%) indique le taux de conversion de l'ester carbonique de glycérol (cyclique) α/α'-acylé de départ. Les valeurs de masse molaire apparente sont obtenues par analyse du milieu de réaction en chromatographie par perméation de gel.

Il va de soi que l'invention peut faire l'objet de nombreuses variantes de réalisation et applications. En particulier le procédé de synthèse des esters carboniques linéaires α/α'-acylés est sujet à des infinités de variantes, en particulier concernant la température de réaction, la pression de réaction, la proportion massique de catalyseur métallique, d'ester(s) carbonique(s) de glycérol (cycliques) α/α'-acylés et d'amorceur organique.

Bien entendu, cette description est donnée à titre d'exemple illustratif uniquement et l'homme du métier pourra y apporter de nombreuses modifications, variantes et applications sans sortir de la portée de l'invention.

## Revendications

1. Oligomères organiques de glycérol acylés de formule (I) générale suivante : 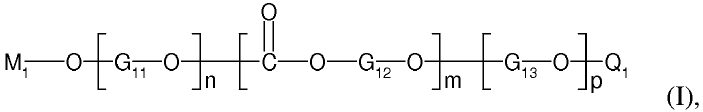 dans laquelle :
- M₁ est un groupement organique choisi dans le groupe formé des groupements de formules suivantes : 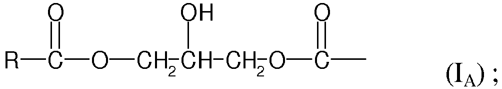 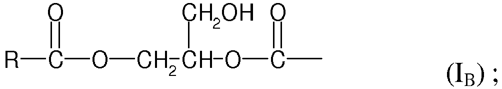 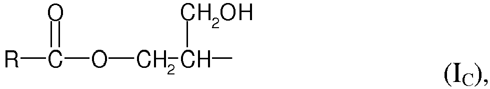 et; 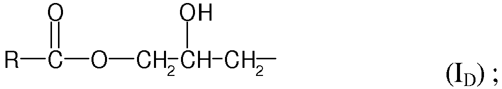 dans lesquels R est un groupement hydrocarboné -notamment un groupement hydrocarboné saturé, un groupement hydrocarboné insaturé ou un groupement hydrocarboné ramifié- comprenant de 1 à 21 atomes de carbone, et ;
- G₁₁ est choisi dans le groupe formé des groupements propyles hydroxylés de formules générales (II_{A}) et (II_{B}) suivantes : 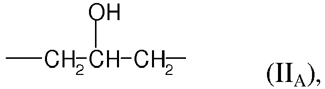 et ; 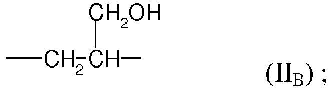
- G₁₂ et G₁₃ sont des groupements propyle α/α'-hydroxy-acylés de formule générale (III) suivante : 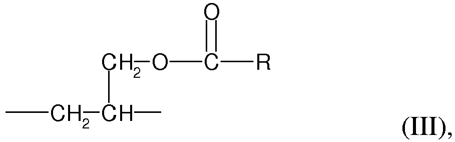
et ;
- Q₁ est choisi dans le groupe formé de l'hydrogène et des groupements organiques formés d'au moins deux atomes liés par des liaisons covalentes et appartenant au groupe formé du carbone (C), de l'hydrogène (H) et de l'oxygène (O);
- n est un nombre entier naturel inférieur à 9 tel que si n = 0 et m ≠ 0, alors M₁ est choisi dans le groupe formé de I_{C} et I_{D}, et ;
- m est un nombre entier naturel inférieur à 5 -notamment de l'intervalle [0 ; 4], et ;
- p est un nombre entier naturel inférieur à 4.

2. Oligomère selon la revendication 1, **caractérisé en ce que** Q₁ est choisi dans le groupe formé de l'hydrogène (H), des groupements propyles hydroxylés de formule (III_{A}) suivante : 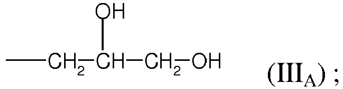 des groupements propyles hydroxylés de formule (III_{B}) suivante : 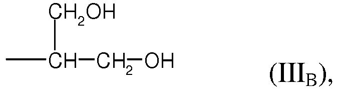 et; des groupements de formules (I_{A}), (I_{B}), (I_{C}) et (I_{D}).

3. Oligomère selon l'une des revendications 1 ou 2, **caractérisé en ce qu'**il est de formule (IV) générale suivante : 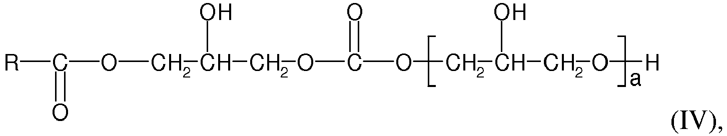 dans laquelle a est un nombre entier non nul inférieur à 9.

4. Oligomère selon l'une des revendications 1 ou 2, **caractérisé en ce qu'**il est de formule (V) générale suivante : 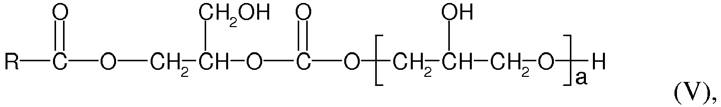 dans laquelle a est un nombre entier non nul inférieur à 9.

5. Oligomère selon l'une des revendications 1 ou 2, **caractérisé en ce qu'**il est de formule (VI) générale suivante : 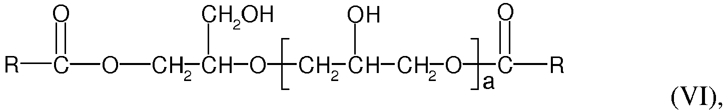 dans laquelle a est un nombre entier non nul inférieur à 4.

6. Oligomère selon l'une des revendications 1 ou 2, **caractérisé en ce qu'**il est de formule (VII) générale suivante : 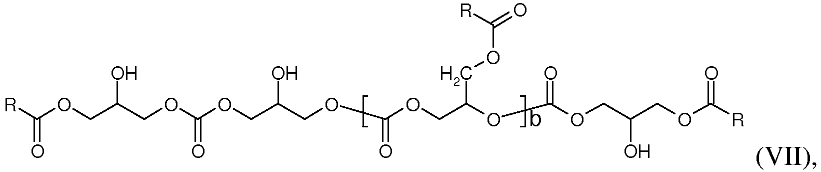 dans laquelle b est un nombre entier non nul inférieur à 5.

7. Oligomère selon l'une des revendications 1 ou 2, **caractérisé en ce qu'**il est de formule (VIII) générale suivante : 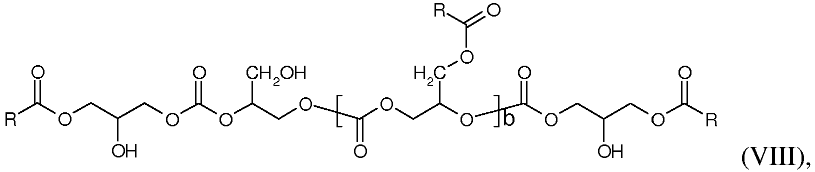 dans laquelle b est un nombre entier non nul inférieur à 5.

8. Oligomère selon l'une des revendications 1 ou 2, **caractérisé en ce qu'**il est de formule (IX) générale suivante : 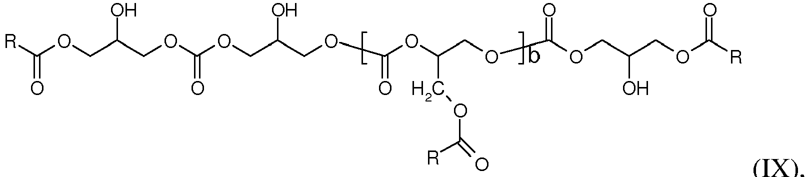 dans laquelle b est un nombre entier non nul inférieur à 5.

9. Oligomère selon l'une des revendications 1 ou 2, **caractérisé en ce qu'**il est de formule (X) générale suivante : 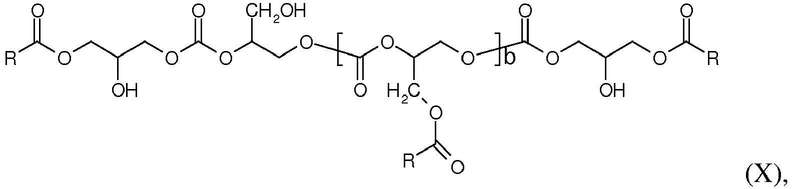 dans laquelle b est un nombre entier non nul inférieur à 5.

10. Oligomère selon l'une des revendications 1 ou 2, **caractérisé en ce qu'**il est de formule (XI) générale suivante : 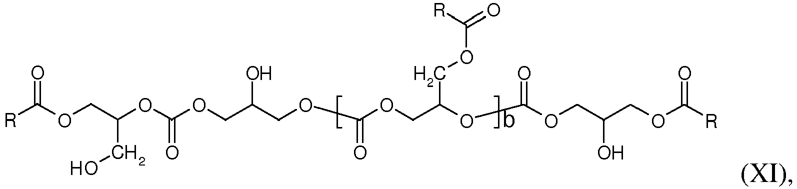 dans laquelle b est un nombre entier non nul inférieur à 5.

11. Oligomère selon l'une des revendications 1 ou 2, **caractérisé en ce qu'**il est de formule (XII) générale suivante : 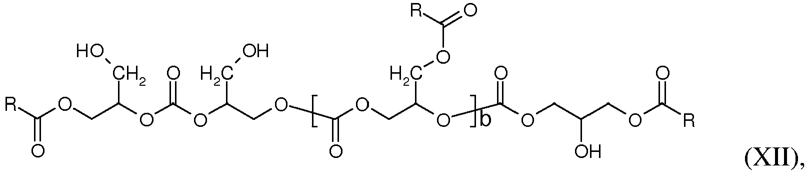 dans laquelle b est un nombre entier non nul inférieur à 5.

12. Oligomère selon l'une des revendications 1 ou 2, **caractérisé en ce qu'**il est de formule (XIII) générale suivante : 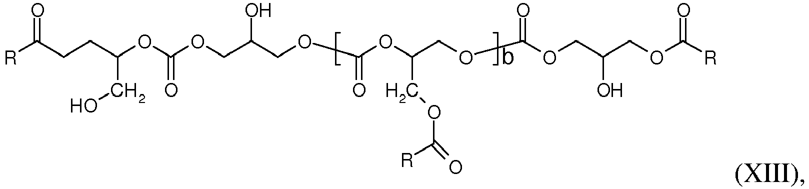 dans laquelle b est un nombre entier non nul inférieur à 5.

13. Oligomère selon l'une des revendications 1 ou 2, **caractérisé en ce qu'**il est de formule (XIV) générale suivante : 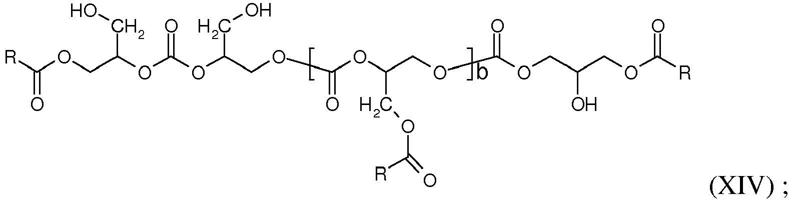 dans laquelle b est un nombre entier non nul inférieur à 5.

14. Oligomère selon l'une des revendications 1 à 13, **caractérisé en ce que** R est un groupement hydrocarboné choisi dans le groupe formé :
o des groupements hydrocarbonés saturés de formule générale -C_{q}H_{2q}+1 dans laquelle q est un nombre entier compris entre 1 et 21, et ;
o des groupements hydrocarbonés insaturés -notamment des groupements hydrocarbonés mono-insaturés de formule générale -CₛH₂ₛ₋₁ dans laquelle s est un nombre entier compris entre 1 et 21, et des groupements hydrocarbonés poly-insaturés-.

15. Oligomère selon l'une des revendications 1 à 14, **caractérisé en ce que** R est un groupement hydrocarboné choisi dans le groupe formé du méthyle (-CH₃), de l'éthyle (-CH₂-CH₃), du *n*-propyle (-CH₂-CH₂-CH₃), de l'*iso*-propyle (-CH(CH₃)₂), du *n*-butyle (-CH₂-CH₂-CH₂-CH₃), de l'*iso*-butyle (-CH₂-CH(CH₃)₂), du *tertio*-butyle (-C(CH₃)₃), du *n*-pentyle (-CH₂-(CH₂)₃-CH₃), de l'hexyle (-(CH₂)₅-CH₃), de l'octyle (-(CH₂)₇-CH₃), de l'undécyle (-(CH₂)₁₀-CH₃), du penta-décyle (-(CH₂)₁₄-CH₃), de l'hepta-décyle (-(CH₂)₁₆-CH₃), du 9-ène-decyle (-CH=CH-(CH₂)₇-CH₃) et du 9-ène-heptadecyle (-(CH₂)₇-CH=CH-(CH₂)₇-CH₃).

16. Composition organique liquide comprenant au moins un oligomère organique de glycérol acylé selon l'une des revendications 1 à 15.

17. Composition selon la revendication 16, **caractérisée en ce qu'**elle comprend une pluralité d'oligomères organiques de glycérol acylés selon l'une des revendications 1 à 15.

## Patentansprüche

1. Organische Oligomere aus acyliertem Glycerol mit der folgenden allgemeinen Formel (I): 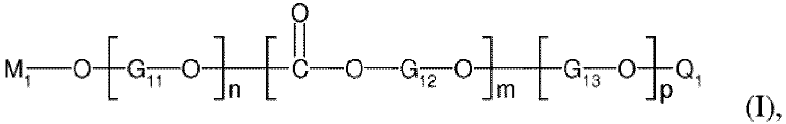 wobei:
- M₁ eine organische Gruppierung ist, ausgewählt aus der Gruppe, gebildet aus den Gruppierungen mit den folgenden Formeln: 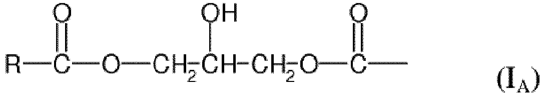 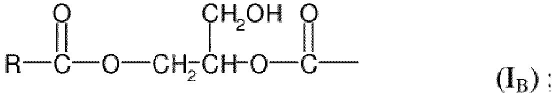 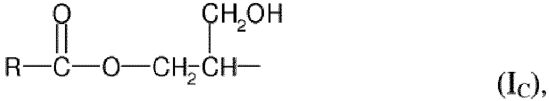 und 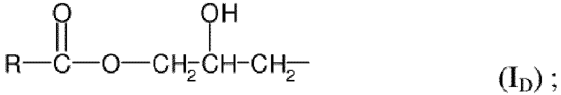 wobei R eine Kohlenwasserstoff-Gruppierung ist - insbesondere eine gesättigte Kohlenwasserstoff-Gruppierung, eine nicht gesättigte Kohlenwasserstoff-Gruppierung oder eine verzweigte Kohlenwasserstoff-Gruppierung - umfassend 1 bis 21 Kohlenstoffatome, und;
- G₁₁ ausgewählt ist aus der Gruppe, gebildet aus hydroxylierten Propylgruppierungen mit den folgenden allgemeinen Formeln (II_{A}) und II_{B}) : 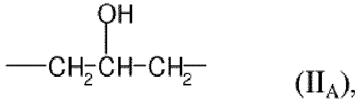 und; 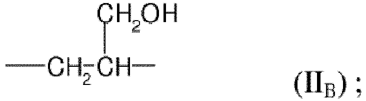
- G₁₂ und G₁₃ α/α'-hydroxy-acylierte Propylgruppierungen mit der folgenden allgemeinen Formel (III) sind: 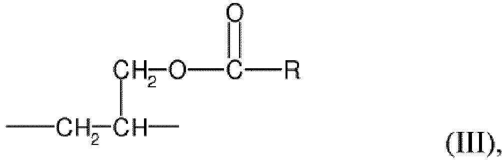 und;
- Q₁ ausgewählt ist aus der Gruppe, gebildet aus Wasserstoff und organischen Gruppierungen, gebildet aus mindestens zwei Atomen, die durch kovalente Bindungen gebunden sind und Teil der Gruppe sind, gebildet aus Kohlenstoff (C), Wasserstoff (H) und Sauerstoff (0);
- n eine natürliche ganze Zahl kleiner als 9 ist, so dass, wenn n = 0 und m ≠ 0, M₁ ausgewählt ist aus der Gruppe, gebildet aus I_{C} und I_{D}, und;
- m eine natürliche ganze Zahl kleiner als 5 ist, - insbesondere des Intervalls [0; 4], und;
- p eine natürliche ganze Zahl kleiner als 4 ist.

2. Oligomer nach Anspruch 1, **dadurch gekennzeichnet, dass** Q₁ ausgewählt ist aus der Gruppe, gebildet aus Wasserstoff (H) hydroxylierten Propylgruppierungen mit der folgenden Formel (III_{A}): 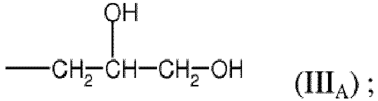 hydroxylierten Propylgruppierungen mit der folgenden Formel (III_{B}) : 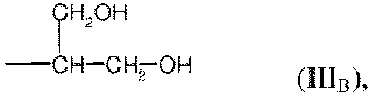 und; Gruppierungen mit den Formeln (I_{A}), (I_{B}), (I_{C}) und (I_{D}).

3. Oligomer nach einem der Ansprüche 1 oder 2, **dadurch gekennzeichnet, dass** es die folgende allgemeine Formel (IV) aufweist: 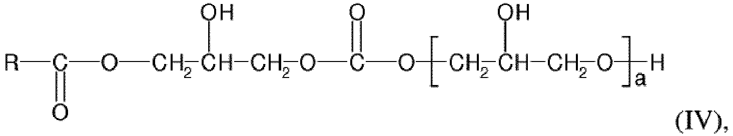 wobei a eine ganze Zahl ungleich Null kleiner als 9 ist.

4. Oligomer nach einem der Ansprüche 1 oder 2, **dadurch gekennzeichnet, dass** es die folgende allgemeine Formel (V) aufweist: 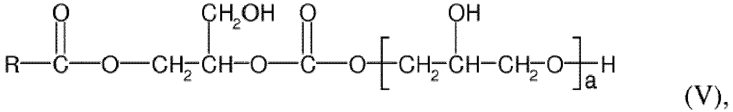 wobei a eine ganze Zahl ungleich Null kleiner als 9 ist.

5. Oligomer nach einem der Ansprüche 1 oder 2, **dadurch gekennzeichnet, dass** es die folgende allgemeine Formel (VI) aufweist: 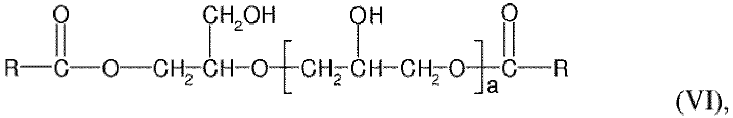 wobei a eine ganze Zahl ungleich Null kleiner als 4 ist.

6. Oligomer nach einem der Ansprüche 1 oder 2, **dadurch gekennzeichnet, dass** es die folgende allgemeine Formel (VII) aufweist: 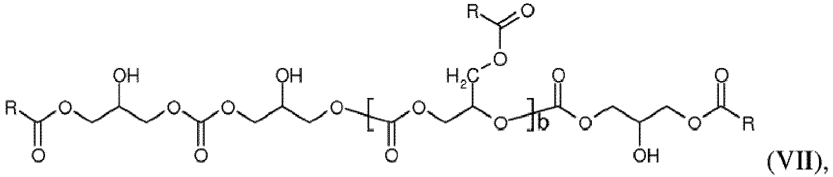 wobei b eine ganze Zahl ungleich Null kleiner als 5 ist.

7. Oligomer nach einem der Ansprüche 1 oder 2, **dadurch gekennzeichnet, dass** es die folgende allgemeine Formel (VIII) aufweist: 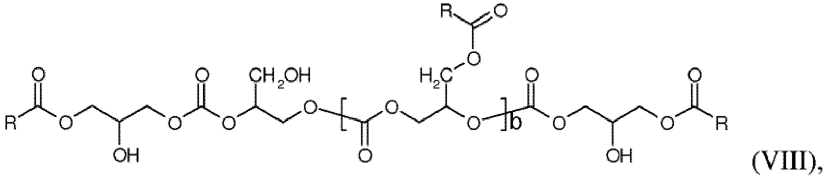 wobei b eine ganze Zahl ungleich Null kleiner als 5 ist.

8. Oligomer nach einem der Ansprüche 1 oder 2, **dadurch gekennzeichnet, dass** es die folgende allgemeine Formel (IX) aufweist : 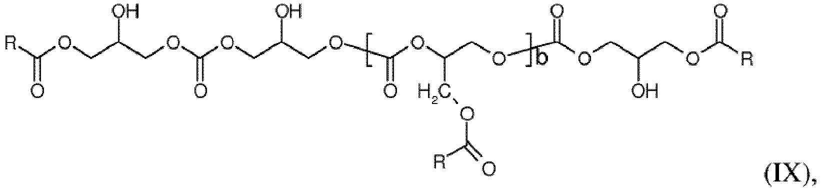 wobei b eine ganze Zahl ungleich Null kleiner als 5 ist.

9. Oligomer nach einem der Ansprüche 1 oder 2, **dadurch gekennzeichnet, dass** es die folgende allgemeine Formel (X) aufweist: 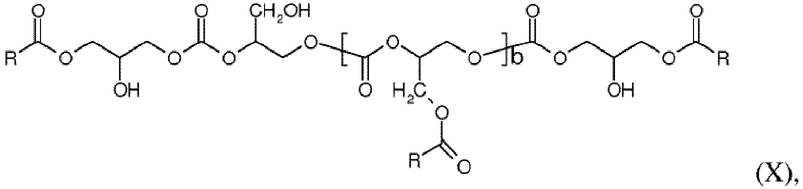 wobei b eine ganze Zahl ungleich Null kleiner als 5 ist.

10. Oligomer nach einem der Ansprüche 1 oder 2, **dadurch gekennzeichnet, dass** es die folgende allgemeine Formel (XI) aufweist : 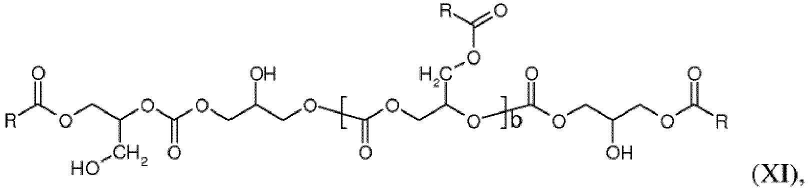 wobei b eine ganze Zahl ungleich Null kleiner als 5 ist.

11. Oligomer nach einem der Ansprüche 1 oder 2, **dadurch gekennzeichnet, dass** es die folgende allgemeine Formel (XII) aufweist : 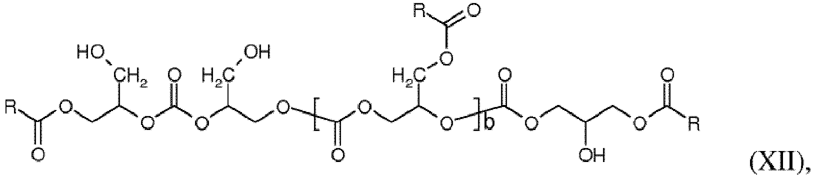 wobei b eine ganze Zahl ungleich Null kleiner als 5 ist.

12. Oligomer nach einem der Ansprüche 1 oder 2, **dadurch gekennzeichnet, dass** es die folgende allgemeine Formel (XIII) aufweist : 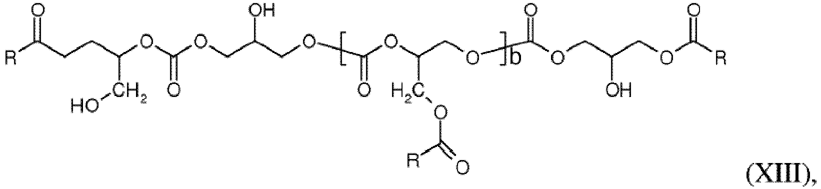 wobei b eine ganze Zahl ungleich Null kleiner als 5 ist.

13. Oligomer nach einem der Ansprüche 1 oder 2, **dadurch gekennzeichnet, dass** es die folgende allgemeine Formel (XIV) aufweist: 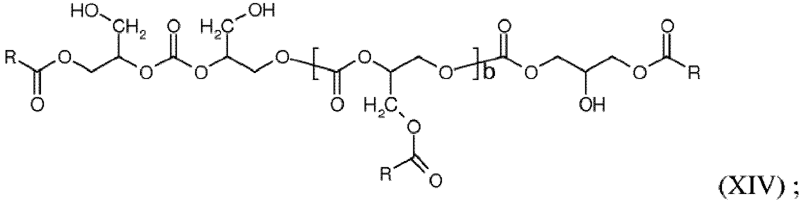 wobei b eine ganze Zahl ungleich Null kleiner als 5 ist.

14. Oligomer nach einem der Ansprüche 1 bis 13, **dadurch gekennzeichnet, dass** R eine Kohlenwasserstoff-Gruppierung ist, ausgewählt aus der Gruppe, gebildet aus:
∘ gesättigten Kohlenwasserstoffgruppen mit der allgemeinen Formel -C_{q}H_{2q}+1, wobei q eine ganze Zahl zwischen 1 und 21 ist, und;
∘ ungesättigte Kohlenwasserstoff-Gruppierungen - insbesondere einfach ungesättigte Kohlenwasserstoff-Gruppierungen mit der allgemeinen Formel -CₛH₂ₛ₋₁, wobei s eine ganze Zahl zwischen 1 und 21 ist, und mehrfach ungesättigten Kohlenwasserstoff-Gruppierungen-.

15. Oligomer nach einem der Ansprüche 1 bis 14, **dadurch gekennzeichnet, dass** R eine Kohlenwasserstoff-Gruppierung ist, ausgewählt aus der Gruppe, gebildet aus Methyl (-CH₃), Ethyl (-CH₂-CH₃), *n*-Propyl (-CH₂-CH₂-CH₃), *iso*-Propyl (-CH(CH₃)₂), *n*-Butyl (-CH₂-CH₂-CH₂-CH₃), *iso*-Butyl (-CH₂-CH(CH₃)₂), *tertio*-Butyl (-C(CH₃)₃), *n-*Pentyl (-CH₂-(CH₂)₃-CH₃), Hexyl (-(CH₂)₅-CH₃), Octyl (-(CH₂)₇-CH₃), Undecyl (-(CH₂)₁₀-CH₃), Pentadecyl (-(CH₂)₁₄-CH₃), Heptadecyl (- (CH₂)₁₆-CH₃), 9-en-Decyl (-CH=CH-(CH₂)₇-CH₃) und 9-en-Heptadecyl (-(CH₂)₇-CH=CH-(CH₂)₇-CH₃).

16. Flüssige organische Zusammensetzung, umfassend mindestens ein organisches Oligomer aus acyliertem Glycerol nach einem der Ansprüche 1 bis 15.

17. Zusammensetzung nach Anspruch 16, **dadurch gekennzeichnet, dass** sie eine Vielzahl von organischen Oligomeren aus acyliertem Glycerol nach einem der Ansprüche 1 bis 15 umfasst.

## Claims

1. An acylated organic glycerol oligomer of the following general formula (I): 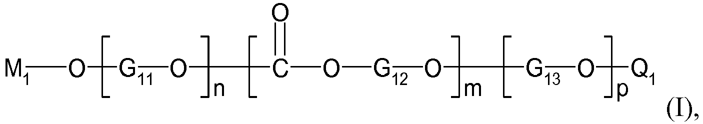 wherein:
- M₁ is an organic group chosen from the group formed of the groups of the following formulae: 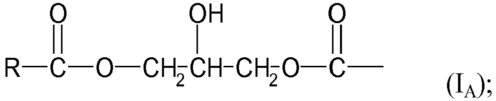 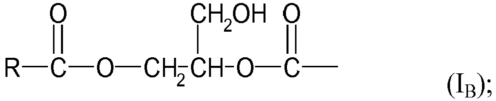 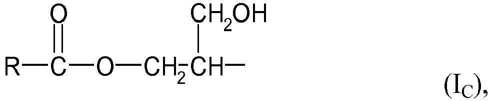 and; 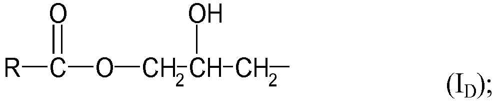
wherein R is a hydrocarbon group - especially a saturated hydrocarbon group, an unsaturated hydrocarbon group or a branched hydrocarbon group - having from 1 to 21 carbon atoms, and;
- G₁₁ is chosen from the group formed of the hydroxylated propyl groups of the following general formulae (II_{A}) and (II_{B}): 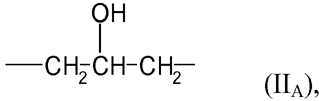 and; 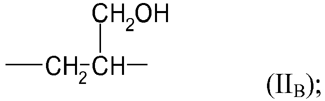
- G₁₂ and G₁₃ are α/α'-hydroxyacylated propyl groups of the following general formula (III): 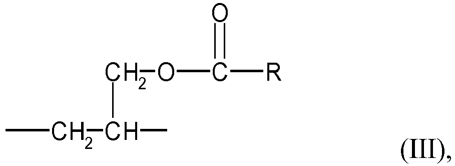 and;
- Q₁ is chosen from the group formed of hydrogen and organic groups formed of at least two atoms bonded by covalent bonds and belonging to the group formed of carbon (C), hydrogen (H) and oxygen (O);
- n is a natural integer less than 9 such that if n = 0 and m ≠ 0, then M₁ is chosen from the group formed of I_{C} and I_{D}, and;
- m is a natural integer less than 5 - especially of the interval [0 ; 4], and;
- p is a natural integer less than 4.

2. The oligomer as claimed in claim 1, **characterised in that** Q₁ is chosen from the group formed of hydrogen (H), hydroxylated propyl groups of the following formula (III_{A}): 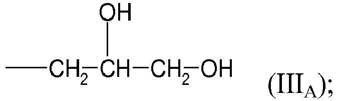 hydroxylated propyl groups of the following formula (III_{B}): 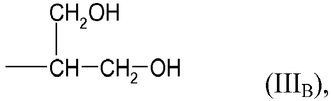 and; groups of formulae (I_{A}), (I_{B}), (I_{C}) and (I_{D}).

3. The oligomer as claimed in either claim 1 or claim 2, **characterised in that** it has the following general formula (IV): 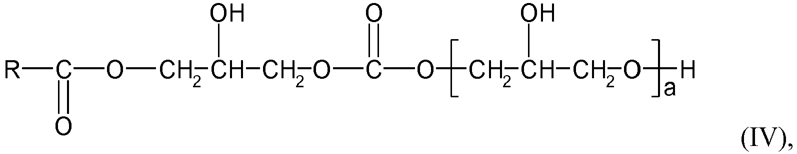 wherein a is a non-zero integer less than 9.

4. The oligomer as claimed in either claim 1 or claim 2, **characterised in that** it has the following general formula (V): 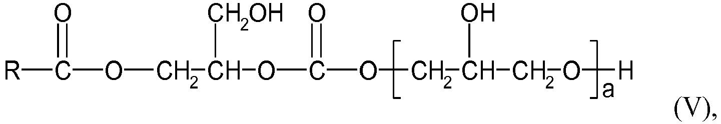 wherein a is a non-zero integer less than 9.

5. The oligomer as claimed in claim 1 or claim 2, **characterised in that** it has the following general formula (VI): 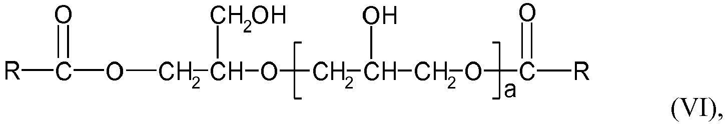 wherein a is a non-zero integer less than 4.

6. The oligomer as claimed in claim 1 or 2, **characterised in that** it has the following general formula (VII): 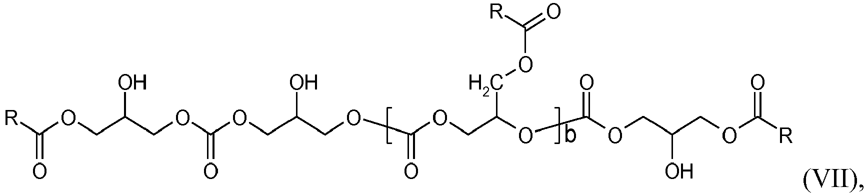 wherein b is a non-zero integer less than 5.

7. The oligomer as claimed in either claim 1 or claim 2, **characterised in that** it has the following general formula (VIII): 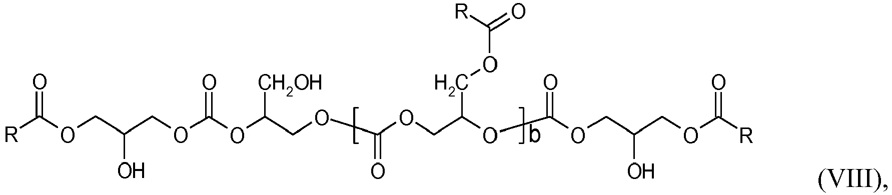 wherein b is a non-zero integer less than 5.

8. The oligomer as claimed in either claim 1 or claim 2, **characterised in that** it has the following general formula (IX): 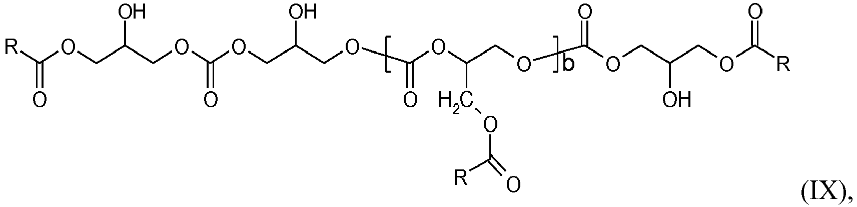 wherein b is a non-zero integer less than 5.

9. The oligomer as claimed in either claim 1 or claim 2, **characterised in that** it has the following general formula (X): 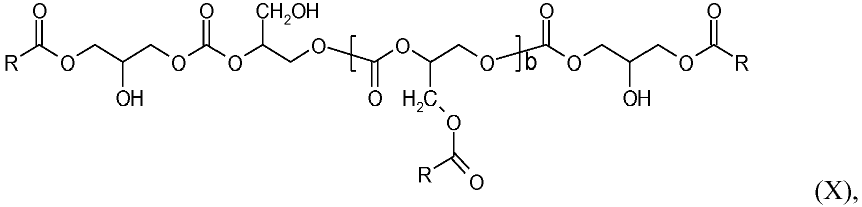 wherein b is a non-zero integer less than 5.

10. The oligomer as claimed in either claim 1 or claim 2, **characterised in that** it has the following general formula (XI): 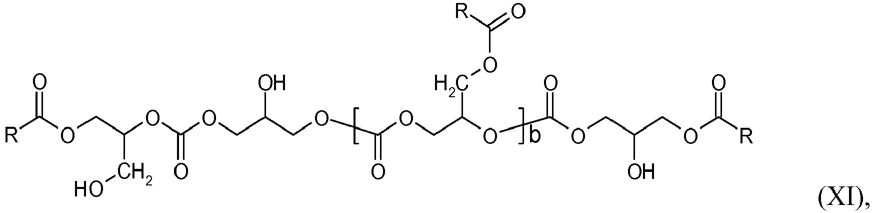 wherein b is a non-zero integer less than 5.

11. The oligomer as claimed in either claim 1 or claim 2, **characterised in that** it has the following general formula (XII): 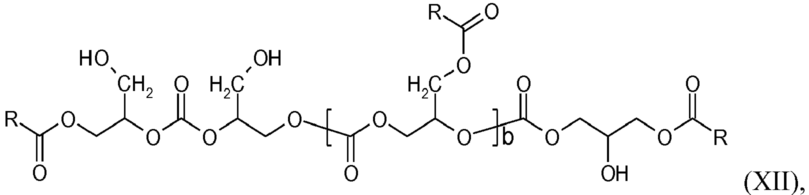 wherein b is a non-zero integer less than 5.

12. The oligomer as claimed in either claim 1 or claim 2, **characterised in that** it has the following general formula (XIII): 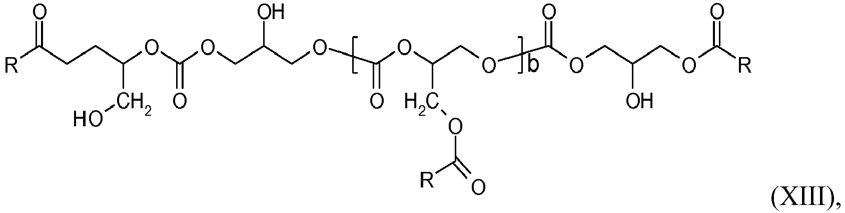 wherein b is a non-zero integer less than 5.

13. The oligomer as claimed in either claim 1 or claim 2, **characterised in that** it has the following general formula (XIV): 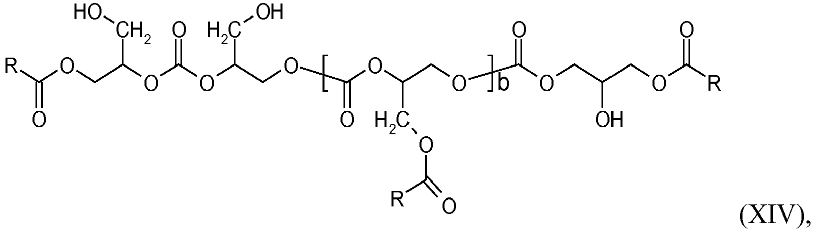 wherein b is a non-zero integer less than 5.

14. The oligomer as claimed in any one of claims 1 to 13, **characterised in that** R is a hydrocarbon group chosen from the group formed of:
o saturated hydrocarbon groups of the general formula -C_{q}H_{2q}+1 wherein q is an integer from 1 to 21, and ;
o unsaturated hydrocarbon groups - especially monounsaturated hydrocarbon groups of the general formula-CₛH₂ₛ₋₁ wherein s is an integer from 1 to 21, and polyunsaturated hydrocarbon groups-.

15. The oligomer as claimed in any one of claims 1 to 14, **characterised in that** R is a hydrocarbon group chosen from the group formed of methyl (-CH₃), ethyl (-CH₂-CH₃), *n*-propyl (-CH₂-CH₂-CH₃), *iso*-propyl (-CH(CH₃)₂), *n*-butyl (-CH₂-CH₂-CH₂-CH₃), *iso*-butyl (-CH₂-CH(CH₃)₂), *tert*-butyl (-C(CH₃)₃), *n*-pentyl (-CH₂-(CH₂)₃-CH₃), hexyl (-(CH₂)₅-CH₃), octyl (-(CH₂)₇-CH₃), undecyl (-(CH₂)₁₀-CH₃), pentadecyl (-(CH₂)₁₄-CH₃), heptadecyl (-(CH₂)₁₆-CH₃), 9-ene-decyl (-CH=CH-(CH₂)₇-CH₃) and 9-ene-heptadecyl (-(CH₂)₇-CH=CH-(CH₂)₇-CH₃).

16. A liquid organic composition comprising at least one acylated organic glycerol oligomer as claimed in any one of claims 1 to 15.

17. The composition as claimed in claim 16, **characterised in that** it comprises a plurality of acylated organic glycerol oligomers as claimed in any one of claims 1 to 15.
